# EUROPEAN PATENT SPECIFICATION

(11) **EP 1 776 921 B1**
(45) Date of publication and mention of the grant of the patent: **12.06.2019**
(21) Application number: 06021656.1
(22) Date of filing: 16.10.2006
(51) Int. Cl.: A61B 5/0215, A61B 5/028, A61M 5/168, F16K 11/044, F16K 15/02

(54) **Device for injectate duration measurement and temperature measurement**
Vorrichtung zum Messen von Injektionsmitteldauer und Temperatur
Dispositif de mesure d'une durée d'une injection et de mesure de température

(30) Priority: 19.10.2005 DE 102005050142
(43) Date of publication of application: 25.04.2007
(73) Proprietor: IPRM Intellectual Property Rights Management AG, 6304 Zug (CH)
(72) Inventor: Pfeiffer, Ulrich, Dr., 81667 München (DE); Moulas, Daniel, 83539 Pfaffing (DE); Knoll, Reinhold, 81543 München (DE)
(74) Representative: Eisenführ Speiser

(56) References cited:
- EP-A- 0 226 220
- EP-A2- 0 900 545
- WO-A1-97/27802
- WO-A2-01/37915
- AU-B2- 418 960
- US-A- 1 686 310
- US-A- 3 834 372
- US-A- 4 013 088
- US-A- 5 148 811
- US-A- 5 235 324
- US-A- 5 354 272
- US-A- 6 089 103
- US-A1- 2003 135 165
- US-A1- 2004 220 542
- US-B1- 6 290 681
- US-B1- 6 491 640
- Grolius, H.-W: "Grundlagen der Hydraulik", 2004, Carl Hanser Verlag, Munich, Vienna pages 135-136,
- "Knaurs Lexikon der Technik", 1988, Droemersche Verlagsanstalt, Munich page 1010,
- Lift, H. u. Hansel, M.: "Hydrauliksysteme in der Bau- und Kommunaltechnik", 1991, Vogel Verlag, Würzburg pages 154, 167-168,

## Description

The invention relates to a device to detect the start and end of injection as well as the injectate temperature during thermodilution determination of cardiocirculatory parameters and intra- and extravascular volumes.

Cardiac Output (CO) and circulatory blood volume are very important parameters to diagnose the condition of clinically ill patients. Measuring these parameters is a very important part of intensive care as well as in medical research. Such measurements are typically performed on critically ill patients in heart surgical treatment and for pharmacological management strategies.

When employing a method of thermodilution for cardiac output determination, a liquid indicator colder than the blood temperature is injected into the right atrium or the superior or inferior vena cava. After a period of time, depending on the blood flow through the heart and pulmonary circulation, a temperature drop can be detected in the femoral artery. By plotting the temperature drop over time, the area under the resulting curve can be used to determine cardiac output. To calculate cardiac output, it is crucial to know the exact temperature of the blood and the injected liquid. The blood temperature can be measured with an indwelling thermistor in the femoral artery, using a sensor that can be a temperature dependent resistor with a negative coefficient (NTC).

The temperature of the injected liquid is also measured with a resistor of the same type. This resistor is in thermal contact with the liquid within the injection channel. Hence, the temperature of the injected liquid can be measured during injection. In order to reuse the sensor, a thermal bridge is established between the sensor and the fluid path using a liquid impermeable material. This device is called IITS device (IITS) and is a sterile disposable item. The IITS is connected in series with the injection channel and contains the holder for the termperature sensor. Additionally, this device includes a membrane as an actuator of a timing device. The defined time intervals on the indicator dilution curve allow calculation of intra- and extravascular volumes between site of injection and site of detection. However, precise detection of the start and finish of the injeciton is necessary.

In US 6,491,640 B1, an injection channel for a blood vessel catheter is described where the start and finish of the injection is carried out by a flow rate switch which is opened or closed if the flow rate in the injection channel exceeds a threshold. In particular, this switch comprises a REED-switch outside the injection channel and a magnet outside the injection channel at a side opposite to the REED-switch, wherein the REED-switch is actuated by the influence of the movement of a movable ferromagnetic member biased by an elastic member in the injection channel which ferromagnetic member is displaced by the influence of the fluid flow inside the injection channel whereby the ferromagnetic member shields the REED-switch from the magnet in a first position and exposes to REED-switch to the magnet in a second position.

The object of the invention was to design an injection channel for a blood vessel catheter which avoids the drawbacks of the prior art and allows for an accurate and automated measurement of the start and finish of the injection.

The object of the invention is achieved by a device as defined in independent claims 1 and 2. The dependent claims are directed to further specific embodiments of the invention. The device according to the invention comprises an injection channel for a blood vessel catheter for injecting an injectate fluid into a blood vessel of a patient for carrying out thermodilution or other dilution measurements or other bolus injections in order to determine hemodynamic parameters of the patient, the injection channel comprising a pressure sensor for sensing the central venous pressure of the patient wherein the pressure sensor is also adapted for sensing a threshold of pressure in the injection channel as instants of begin and end of an injection process.

An injection channel is an arrangement which allows for injecting an injectate fluid into a blood vessel of a patient, especially for carrying out thermo-dilution or dye-dilution measurements in order to determine hemodynamic parameter of the patient. The injection channel is preferably connected to a blood vessel catheter. This blood vessel catheter is applied to a venous system of the patient. Thus, the physiological central venous pressure is applied to the injection channel from the patient. In case that the injection process is in process, the pressure within the injection channel will rise above a certain limit which would not be reached under normal physiological conditions.

The pressure sensor is adapted for sensing the pressure within the injection channel, i.e. the central venous pressure if no injection process takes place. The pressure sensor will measure the central venous pressure of the patient which is in the magnitude of 50 mmHg.

The pressure sensor is also adapted for sensing a threshold of pressure in the injection channel indicating the begin and end of an injection process. Since during the injection process the pressure will be much higher than the central venous pressure of the patient, the reaching of a predefined threshold of pressure in the injection channel will indicate the begin of the injection process. After the injection process is over, the pressure will drop again under a threshold of pressure in the injection channel. Thus, the drop of the pressure will indicate the end of the injection process. Thus, the pressure sensor is adapted for sensing a predefined threshold of pressure thus determining the begin and the end of an injection process. For instance, the physiological central venous pressure of a patient is 50 mmHg. Now, an injection of an injection duration of 5 seconds is applied raising the pressure within the injection channel to 1000 mmmHg. A threshold of pressure is predefined at 300 mmHg. Reaching this threshold will now indicate the begin of the injection process. After the injection process is over afer a duration of 5 seconds, the pressure will drop under the 300 mmHg. This point of time indicates the end of the injection process. Afterwards the pressure sensor again indicates the physiological central venous pressure of 50 mmHg.

Thus, a design of an injection channel is defined which only uses one pressure sensor for both measuring the physiological central venous pressure and indicating the begin and the end of an injection process. With such an injection channel there is no need for additional pressure sensors and devices or other ferro-magnetic devices to measure the begin and the end of an injection process. Thus, a simplified design of an injection channel is provided.

In a further embodiment of the present invention, the injection channel further comprises a protection mechanism associated with the pressure sensor to protect the pressure sensor against damages by high pressure during the injection process.

Advantageously, the pressure sensor comprises a protection mechanism in order to avoid damages by high pressure during the injection process. Since the physiological central venous pressure is much lower than the pressure present during the injection process, the pressure sensor could be damaged when subjected to a much higher pressure than usually sensed by this type of pressure sensor. Thus, it is advantageous to provide a protection mechanism to prevent any damages to such a pressure sensor. With such a protection mechanism, a pressure sensor type can be used which does not have to fullfill the requirement of accuracy over a wide range of a pressure. Thus, this pressure sensor can be chosen to be accurate for the range of the physiological central venous pressure and can be blocked by the protection mechanism in case that the threshold as predefined is exceeded. Since this protection mechanism is associated with the pressure sensor it might be integral with the pressure sensor or provided upstream the pressure sensor to close the injection channel to the pressure sensor in case the threshold is exceeded. Thus, an injection channel design is provided which can make use of a less robust pressure sensor being adapted to accurately measure the physiological central venous pressure.

In a further embodiment of the present invention, the threshold of pressure is sensed by the protection mechanism of the pressure sensor.

In case that a protection mechanism associated with the pressure sensor is provided to prevent damages by high pressure during the injection process it is advantageous to take the point of time of the begin and the end of an injection process from the protection mechanism reaching the predefined threshold. Thus, the activation of the protection mechanism means that the predefined threshold is reached, i.e. the injection process began. Thus, this point of time defines the begin of the injection process. As soon as the threshold is undershot, the protection mechanism is activated. Thus, the release of the protection mechanism indicates the end of the injection process. Thus, the activation and deactivation of the protection mechanism indicates the necessary points of time of the begin and the end of the injection process. Thus, the activation and deactivation of a protection mechanism is directly used to define these points of times.

In a further embodiment of the present invention, the injection channel further comprises a syringe, a first valve, a second valve, a temperature sensor inside the injection channel for sensing the temperature of the injectate fluid passing through the injection channel and a reservoir for injectate medium, wherein from an upstream point to a downstream point the reservoir, the second valve, the first valve and the temperature sensor are connected to each other by a fluid flow means, the syringe is connected to the fluid flow means between the first valve and the second valve and the pressure sensor is arranged downstream the first valve.

With the syringe it is possible to aspirate injectate solution from a reservoir and by pushing the syringe to deliver the injectate to the patient. First and second valves are provided, preferably as check valves which allow flow only in one direction. When arranging first and second valves in one row and connecting the syringe between the first and second valve, aspirating the syringe will open flow in one direction and pushing the syringe plunger will open flow in the other direction. Thus, it is possible to fill the syringe with injectate or any fluid by aspirating the syringe and deliver injectate fluid to the patient by pushing the syringe plunger via the other way now opened by the valves.

Further, a temperature sensor is provided to read the temperature of the medium within the injection channel. Thereby, it is possible to monitor the bolus initiated by the injection process since the temperature of the injectate medium differs from the temperature present in the injection channel.

The reservoir for injectate is connected to the injection channel. This reservoir is arranged at an upstream point to the second valve and the first valve at a downstream point. Thus, it is possible for the syringe placed between the two valves to aspirate injectate from the reservoir and deliver it to the patient by pushing the syringe plunger and thus opening the first valve.

The components of the injection channel are connected by fluid flow means, which are preferably plastic tubes.

In a further embodiment of the present invention, the injection channel further comprises a a first stop cock arranged downstream of all other components adapted to be connected to the catheter.

The first stop cock preferably has three openings of which two can be connected together. Thus, it is possible to open the line from the injection channel to the catheter and thus to a patient or to choose to close this line and to connect further equipment to the third entry of the stop cock.

In a further embodiment of the present invention, the catheter is a single lumen venous catheter.

With the present invention, it is one advantage that the arrangement can be used with a single lumen venous catheter. Thus, it is not necessary to use a multi-lumen catheter which is more difficult and delicate to manufacture and use. Further, the speed of delivery can be better adjusted with a single lumen venous catheter.

Furthermore it is advantageous that, by automating the switching process between injection and infusion through the same catheter lumen, the infusion or flushing process, respectively, is continued without the need of user interaction after commencement of injection and thus the likelihood of thrombus formation at the distal end of the respective lumen is minimized.

In a further embodiment of the present invention, the pressure sensor is additionally connected to the fluid flow means between the reservoir and the second valve.

Preferably, the pressure sensor is connected to the fluid flow means between the reservoir and the second valve via a second stop cock or via an automatic valve or by a further link of the fluid flow means. Thus, it is possible to bypass the pressure sensor during the process of injection. In this way, it is possible to easily design an injection channel arrangement with an effective protection mechanism for the pressure sensor.

In a further embodiment of the present invention, the protection mechanism comprises a check valve (5) closed by default and a check valve (13) open by default.

With such an arrangement it is possible to protect the pressure sensor during the process of injection. The check valve closed by default can be the first valve described above. Preferably, the check valve open by default is a further check valve situated next to the pressure sensor. In case of the syringe being sucked to load with injectate fluid, the check valve closed by default is closed and allows the syringe to be filled. During the injection process the syringe is pushed thus the check valve closed by default is now opened. Since the pressure is now actuating on the check valve which is open by default situated next to the pressure sensor, this check valve will close and thus protect the pressure sensor.

In a further embodiment of the present invention, the protection mechanism comprises a check valve (5) closed by default and a protection valve (14) which closes at over pressure.

In this arrangement, the protection valve (14) is situated next to the pressure sensor and will close at over pressure. This is the case during the injection process when the check valve (5) which is closed by default will open due to the pressure applied by the pushing syringe. In this case, the protection valve (14) will close due to over pressure and thus protect the pressure sensor situated behind the protection valve (14).

In a further embodiment of the present invention, the protection mechanism (10) comprises an automatic valve (12).

With this automatic valve (12) it is possible to integrally combine the check valve (5) closed by default and a protection mechanism as a check valve or a protection valve (14). With the automatic valve a device is provided which will open the path from the syringe to the catheter during the injection process and, at the same time, block flow to the pressure sensor. Thus, the pressure sensor is protected during the injection process.

The object of the present invention is also achieved by an automatic valve (12) for use in an injection channel as described above comprising a first opening (21) to the injectate fluid, a second opening (22) to the venous catheter (1) a third opening (23) to the pressure sensor (4), a sealing matter(25,26) being movable between the first opening (21) and the third opening (23) and a spring (24) extending to the first opening (21) and pressing the sealing matter (25,26) against the first opening (21) thereby closing said first opening (21), wherein upon a differential pressure over a predefined threshold being applied onto the first opening (21) by the injectate fluid the sealing matter (25,26) is pressed against the third opening (23) thereby closing said third opening (23) and giving free the first opening (21). With this automatic valve an integral solution is provided to protect the pressure sensor against damages due to high pressure during the injection process. With the three openings the automatic valve resembles a stop cock which allows flow between the second opening 22 and the third opening 23 under normal conditions. Thus, the catheter is opened to the pressure sensor. Thus, the pressure sensor records the central venous pressure of the patient. During the process of injection, the syringe pushes injection fluid through the first opening. Thereby, the sealing matter is moved and closes the third opening thus giving free a channel between the first opening and the second opening. Thus, the injectate fluid is delivered to the patient via the catheter.

Since the sealing matter (25,26) is movable between the first and the third opening, it alternatively blocks the path between the second opening and the third opening or the first opening, respectively. The spring (24) pushes the sealing matter (25,26) against the first opening. Thus, the default position of the sealing matter (25,26) is to block the first opening and to give a path between the second and the third opening.

Upon a pressure over a predefined threshold being applied onto the first opening the sealing matter will move against the spring and block the third opening thus giving free a passage between the first opening and the second opening. By choosing the strength of the spring a threshold can be predefined which has to be applied to block the third opening. Thus, a pressure can be predefined which could harm the pressure sensor when exceeded.

The invention is now described with respect to the figures in which advantageous embodiments are displayed.
- Fig. 1: is a schematic view of one embodiment of an injection channel according to the present invention with a stop cock;
- Fig. 2: is a schematic view of an injection channel of the present invention with an in line design;
- Fig. 3: is a third embodiment of the injection channel according to the present invention with an automatic valve;
- Fig. 4: is a schematic view of an injection channel according to the present invention with a check valve open by default;
- Fig. 5: is a schematic view of a fifth embodiment of the present invention with a protection valve and
- Fig. 6: is cross-sectional view of an automatic valve according to one embodiment of the present invention.
- Figure 7: is a cross-sectional view of an automatic valve according to one embodiment of the present invention.

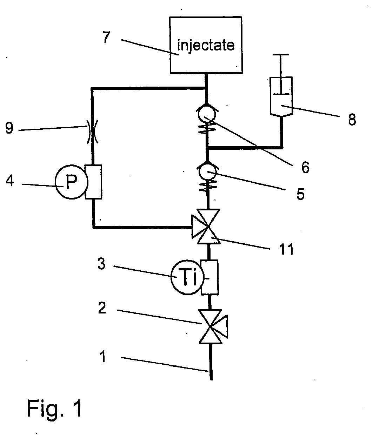

**Figure 1** shows a schematic view of one embodiment of an injection channel according to the present invention with a stop cock 11. An injectate reservoir 7 is connected via a tube to the second valve 6 and the first valve 5 both of which are check valves open in one direction when pressure is applied. Between both valves 5 and 6 a syringe 8 is connected to the injection channel. Downstream the first check valve 5 a stop cock 11 is arranged. The first check valve 5 is connected with a first opening of this stop cock 11, the second opening is connected to the blood vessel catheter 1 via a temperature sensor 3 and another stop cock 2. The third opening of the stop cock 11 is connected to a pressure sensor 4. The pressure sensor is connected to the injection channel downstream the injectate reservoir 7 and upstream the second valve 6. In between the connection between the pressure sensor and the injection channel there is provided a capillary 9.

Under normal conditions with no injection process running, stop cock (11) is manually switched to establish a path between the blood vessel catheter (1) and the pressure sensor (4). In this state, the pressure sensor will measure the central venous pressure of the patient. In order to prevent catheter blocking by thrombus formation, capillary (9) ensures constant steady flow of for instance 3 ml per hour out of the fluid reservoir (7), which is kept at 300 mmHg by means of a pressurized flush solution/injectate bag as known in the art. The stop cock (2) arranged between the single lumen central venous catheter (1) and the stop cock (11) is intended for blood samples and would normally give free a path between the catheter (1) and stop cock (11).

In case of injection, the stop cock 11 is manually switched from pressure measurement to injection in order to give free the path between catheter 1 and check valve 5, check valve 6 and syringe 8. Thus, when manually switched, stop cock 11 will block the path between the catheter 1 and the pressure sensor 4. Now, the syringe can be pulled. Thereby, the first check valve 5 will close and block the path to the catheter 1 whereas check valve 6 will open due to the negative pressure created by the syringe 8 and allow injectate fluid to fill the syringe 8 from the reservoir 7 via the second check valve 6 into syringe 8. As soon as the syringe 8 is pushed, check valve 5 will open due to the pressure coming from the injection channel out of syringe 8 and check valve 6 will close and block the path to the injectate reservoir 7. Thus, the injectate fluid will be delivered from the syringe 8 via the open check valve 5 and stop cock 11 over the temperature sensor 3 and the stop cock 2 into the catheter 1. The opening of check valve 5 is now an indication that the injection process began. The opening of the check valve 5 will be monitored and recorded as the begin of the injection process. This can be accomplished by a computer (not shown) connected to the first check valve 5. As soon as the injection process stops the first check valve 5 will be closed again by the springlike element pushing the check valve 5 in a closed position. This is due to the fact that the pressure created by the pushing of syringe 8 fell under a certain threshold and therefore check valve 5 closes again. By choosing the strength of the springlike element in check valve 5, this threshold can be predefined. By recording this closing of the check valve 5, the end of the injection process can be monitored. Thus, opening and closing of the first check valve 5 will give the instance of begin and end of the injection process. By manually switching stop cock 11 during the injection process, pressure sensor 4 is protected against over pressure created by the injection process when pushing the syringe 8. After the injection process is finished, stop cock 11 is switched back again to give a path between the pressure sensor 4 and the catheter 1. Thus, the pressure sensor 4 will record the central venous pressure of the patient again.

As a result, an injection channel design is provided which allows for measuring the central venous pressure of the patient with the pressure sensor 4 and has a first check valve 5 associated to this pressure sensor 4 which indicates when a threshold of pressure is exceeded thus giving the instant of begin of an injection process and later when released after the end of the insertion process gives the end of this injection process. With stop cock 11 a protection mechanism is provided for the pressure sensor 4.

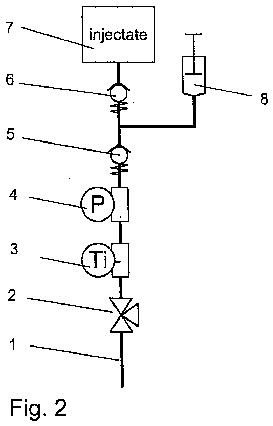

In **Figure 2** a schematic view of an injection channel of the present invention with an inline design is shown. The injectate reservoir is arranged upstream a second check valve 6 and a first check valve 5. Between the first check valve 5 and second check valve 6 a syringe 8 is connected to the injection channel. Downstream the first check valve 5 a pressure sensor 4 and a temperature sensor 3 are provided. A catheter 1 is connected to this injection channel via a stop cock 2. All components are placed straight inline.

In case that no injection process is running stop cock 2 will be opened to the path between catheter 1 and the injection channel comprising the temperature sensor and the pressure sensor 4. Thus, the pressure sensor 4 will give the central venous pressure of the patient. Both check valves 5 and 6 will be closed due to the springlike element integrated in these check valves. In case an injection process is started, syringe 8 will be pulled and the second check valve 6 will open due to the negative pressure created by the syringe 8. Thus, injectate fluid will be aspirated from reservoir 7 into the syringe 8 with check valve 6 being opened and the first check valve 5 still being closed. In the phase that syringe 8 is now pushed, check valve 6 will close and the first check valve 5 will open due to the pressure created by the syringe directed into the injection channel. Thus, the pressure sensor 4 will now monitor an increase in pressure. This pressure raises above a predefined limit (for instance 300 mmHg), which could not be reached under normal physiological conditions. Thus, this event is directly associated with the injection start time. Thus, the reading of the pressure of the pressure sensor 4 above a predefined threshold now indicates the start of the injection process. Thus, the begin of the injection process is defined and can be processed by a computer (not shown) connected to pressure sensor 4. The temperature sensor then reads the change in temperature as known in the state of the art. Since stop cock 2 gives path between the syringe and catheter 1, the injectate fluid will be delivered to the patient via catheter 1. After the injection process is finished, the pressure created by the syringe 8 will fall under the predefined threshold and check valve 5 will close again. As a result, the pressure read by the pressure sensor 4 has now droped under the predefined threshold thus indicating the end of the injection process. Thus, an automated measurement of begin and end of the injection process is achieved by this injection channel arrangement. Thus, the pressure sensor 4 used for measuring the physiological central venous pressure is used to further read the exceeding of a predefined threshold thus giving the begin and the end of the injection process. The pressure limit value used for a threshold is located between the maximum physiological central venous pressure, i.e. 50 mmHg and delivers pressure which is at least necessary to apply the injection volume in a reasonably maximum injection duration of i.e. 5 seconds. Since this injection line is applied central venous, the pressure sensor is used for measuring the central venous pressure if no injection happens and indicates the instants of begin and end of the injection process. The stop cock 2 again as in figure 1 is used for blood samples and gives free the pathway between the catheter and the injection line.

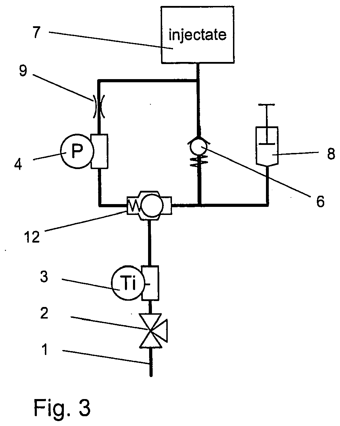

**Figure 3** is a third embodiment of the injection channel according to the present invention with an automatic valve 12.

The design of the injection channel arrangement according to figure 3 corresponds to the arrangement as shown in figure 1 whereas stop cock 11 and the first check valve 5 of figure 1 are replaced by an automatic valve 12 of figure 3. This automatic valve 12 combines the functionality of a check valve and a stop cock. Under normal conditions with no injection process running the automatic valve 12 blocks the way to the syringe 8 and gives path between catheter 1 and the pressure sensor 4. Thus, the pressure sensor 4 reads the physiological central venous pressure of the patient. In case that the injection process begins, syringe 8 is pulled and injectate fluid is sucked from injectate reservoir 7 into the syringe 8 thus opening the second check valve 6. The automatic valve 12 still blocks the way to the syringe 8 due to the springlike element whithin the automatic valve 12 and the negative pressure created by syringe 8 both directed in the same direction. After the syringe 8 is filled the injection process starts by pushing the syringe 8. Thus, the automatic valve 12 will give path between syringe 8 and catheter 1 due to the pressure created by the syringe 8 within the injection channel thus moving for instance a sealing matter in the automatic valve 12. At the same time that the path between syringe 8 and catheter 1 is given free, the path between the pressure sensor 4 and catheter 1 is blocked. This movement of the automatic valve 12 can be monitored by a computer (not shown) connected to the automatic valve thus indicating the begin of the injection process. If the automatic valve 12 is activated, the pressure sensor 4 is disconnected from catheter 1 and its pressure is suddenly raising to the activating threshold. Alternatively or in addition, the reading of the pressure sensor 4 can be used to monitor the increase in pressure over a predefined threshold thus indicating the begin of the injection process. The automatic valve 12 blocks the path between catheter 1 and the pressure sensor 4. Thus, the pressure created by the syringe 8 can not reach the pressure sensor 4. Thereby, the pressure sensor 4 is protected by the automatic valve 12 against damage due to overpressure created by the syringe 8 during the injection process. The pressure sensor monitored an increase of the pressure to a predefined threshold and is then protected by the blocking of the automatic valve 12. When the injection process is over, the pressure from syringe 8 into the injection channel will decrease. As a result, the automatic valve 12 will close the path between the catheter 1 and the syringe 8 again. This moment again is recorded as the end of the injection process. Further, by blocking the path between catheter 1 and syringe 8, automatic valve 12 opens the path between the pressure sensor 4 and the catheter 1. Thus, the pressure sensor will now read again the physiological central venous pressure. With this arrangement, the begin and the end of the injection process can easily and automatically be monitored. At the same time, the automatic valve 12 provides a protection of the pressure sensor 4 against overpressure created by pushing the syringe thus increasing the pressure in the injection channel. Because of the capillary 9, the pressure measured by the pressure sensor 4 during the injection process would drift to the pressure of the fluid reservoir 7, i.e. 300 mmHg. At the end of the injection process the value would return to physiological value. The default position of the automatic valve 12 as well as the threshold at which the automatic valve opens due to the pressure created by the syringe will be set by the strength of the springlike element.

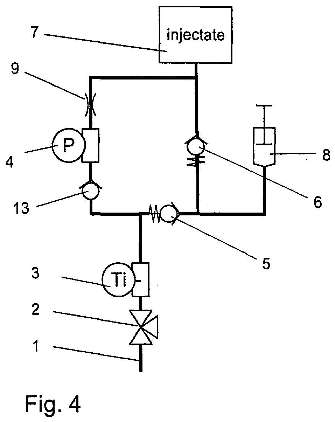

**Figure 4** is a schematic view of an injection channel according to the present invention with check valve opened by default 13.

This arrangement corresponds to the arrangement of figure 1 whereas the stop cock 11 of figure 1 is replaced by a third check valve 13 which would normally be open. The first check valve 5 and the second check valve 6 are directed in the same direction. In between both the first check valve 5 and the second check valve 6 the syringe 8 is connected. The pressure sensor 4 is now protected by the third check valve 13 which is usually open.

Under normal conditions if no injection process is running the first check valve 5 will close the pathway between the catheter and the syringe and thus only the pathway between the catheter 1 and the pressure sensor 4 is open. The third check valve 13 is usually open in this position thus giving free the pathway between catheter 1 and the pressure sensor 4. As a result, the pressure sensor 4 will read the physiological central venous pressure of the patient. In case that the injection process starts, the second check valve 6 will open when syringe 8 is pulled and allow injectate fluid to flow from the reservoir 7 to the syringe 8. The first check valve 5 is still closed in that stage. When the syringe 8 is now pulled to deliver the injectate fluid, the first check valve 5 will open due to the pressure difference created by the pushing of the syringe 8. At the same time the third check valve 13 will - due to the increasing pressure created by the syringe 8 - close and protect the pressure sensor 4 against a damage due to exceeding high pressure created by the syringe 8. As a result, the injectate fluid will be delivered from syringe 8 to catheter 1 whereas the pressure sensor 4 due to the capillary 9 will drift to the pressure present in the injectate reservoir. The instant pressure increase and drifting speed is dependent on the compliance of the fluid system between check valve 13 and capillary 9, which is preferably choosen appropriately. If the system is stiff, then the pressure at sensor 4 raises to the pressure of the fluid reservoir 7 before reversing flow and closing check valve 13 . After the injection process is over and the third check valve 13 is opened again, the pressure sensor 4 will show the central venous pressure of the patient via catheter 1. Again, the injection start and end time can be detected either by the switching of the first check valve 5 and 13 respectively or the pressure sensor 4. Further, the pressure sensor 4 will show a sudden increase and drift behaviour to the injectate reservoir 7 after the third check valve 13 is closed. Thus, also the values of the pressure sensor 4 would give an indication of start and end of the injection process. Since the increase at the start of the injection until the predefined threshold of the check valve 13 is reached gives the start. Further, the drop under this threshold indicates the end of the injection process.

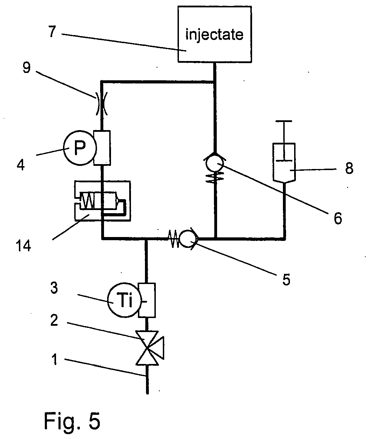

**Figure 5** is a schematic view of a fifth embodiment of the present invention with a protection valve 14.

The arrangement corresponds to the arrangement as described in figure 1 whereas the stop cock 11 is replaced by a tube connection and the protection valve 14.

In this arrangement, the automatic valve function is obtained by the protection valve 14 which closes at over a pressure and a normally closed check valve 5. The protection valve 14 could be implemented by a piston which is kept in open position by a spring and would be closed at excessive pressure. Thus, the opening and closing of the protection valve as well as the respective lines of increase of pressure detected by the pressure sensor 4 could be used as an indication of the begin and the end of the injection process.

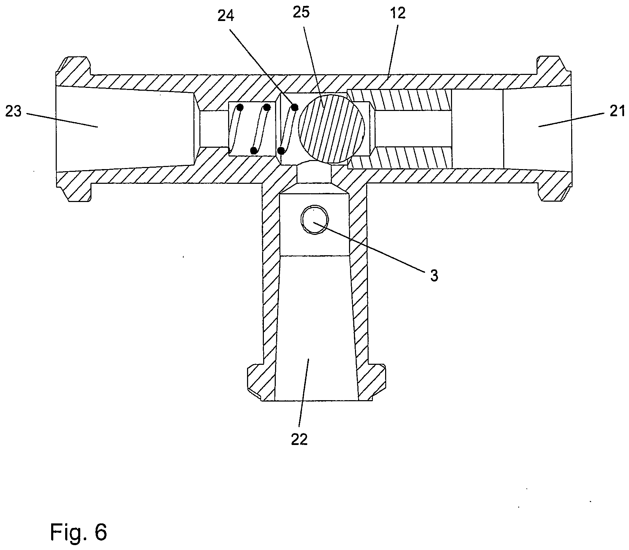

**Figure 6** is a cross-sectional view of an automatic valve according to one embodiment of the present invention.

This automatic valve as shown in figure 6 could be the automatic valve 12 used in the arrangement of figure 3. This automatic valve 12 comprises three openings, i.e. a first opening 21 to which the syringe can be connected, the second opening 22, to which the catheter can be connected and a third opening 23, to which the pressure sensor can be connected. Further, a temperature sensor 3 is provided near the central part of the automatic valve 12 within the pathway of the second opening 22. A ball 25 is provided in such a way that it can toggle between two hubs between the first opening 21 and the third opening 23. A spring 24 is arranged such that it exerts a force against the ball 25 to push it into the hubs directs to the first opening 21 thus closing the path way of the first opening 21 in case no pressure difference is provided between all three openings. The strength of the spring 24 predefines the force necessary to be applied from the direction of the first opening 21 to the automatic valve 12 in order to shift the ball 25 against the spring 24 in order to open the pathway from the first opening 21 to the second opening 22 in the automatic valve 12.

Under normal conditions, if no excessive pressure is applied by the syringe, the first opening 21 is blocked by the ball 25. The pathway between the second opening 22 and the third opening 23 is opened. The spring 24 pushes the ball 25 against a hub thus closing the first opening 21. In case that a pressure is now applied to the first opening 21, the ball 25 is moved against the spring 24 thus giving free the hubs connected to the first opening 21 and at the same time closing the hubs of the third opening 23 thus blocking third opening 23 and giving a path between the first opening 21 and the second opening 22. If the pressure applied to the first opening 21 is slowly increased, there will be a stage where the ball 25 is moved against the spring 24 and somewhat opens the hub connected to the first opening 21 but when the ball 25 not fully closes the pathway to the third opening 23. In this stage the pressure sensor connected to the third opening 23 will detect an increase in a pressure. After the pressure increased such that the ball 25 now is pressed against the strength of spring 24 against the hubs connected to the third opening 23, the pressure sensed by the pressure sensor will rest at a certain plateau and slowly drift to the pressure of the injectate reservoir due to the use of capillary 9 (see figure 3). Thus, the increase of the pressure sensor 4 as monitored by a computer (not shown) clearly indicates the begin of the injection process. After the end of the injection process, the spring 24 will again push the ball 25 into the hubs associated to the first opening 21 thus blocking the pathway to the first opening 21 and giving free the pathway between the second opening 22 and the third opening 23. Now, the pressure drops back to the central venous pressure value. This drop in pressure monitored by a pressure sensor 4 will now indicate the point of time at which the injection process ended.

With the automatic valve 12 as shown in figure 6, a compact and integral design is provided for automatically measure the instants of begin and end of the injection process using the pressure sensor 4 which is usually used to measure the physiological central venous pressure at the time, when no injection process is running.

As a result, a safe, cheap and simple solution for simultaneously measuring central venous pressure and performing thermodilution injections and providing an injectate temperature and start time and end time of the injection is provided.

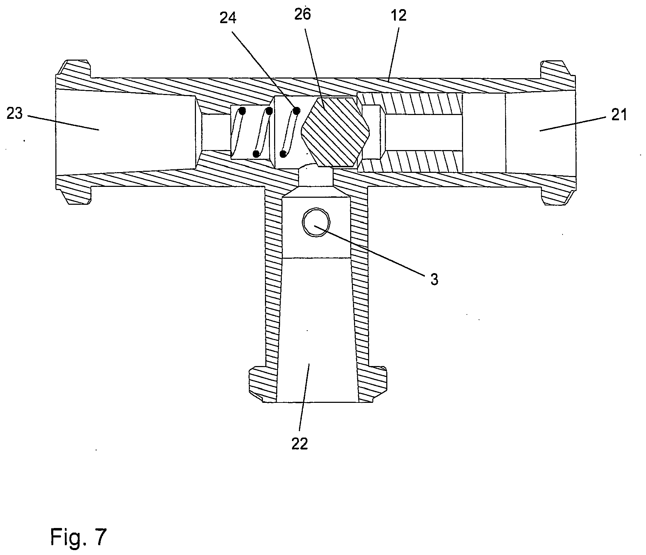

**Figure 7** is a cross-sectional view of an automatic valve according to one embodiment of the present invention.

This automatic valve as shown in figure 7 could be the automatic valve 12 used in the arrangement of figure 3.

It is simmilar to Figure 6 but ball 25 is replaced by a cylindric sealing matter 26. If the automatic valve 12 is activated the sealing matter will break the pathway to the third opening 23 before making the pathway to the first opening 21. The instant pressure increase and drifting speed is dependent on the compliance of the fluid system between automatic valve 12 and capillary 9, which is preferably choosen appropriately. If the system is stiff, then the pressure at sensor 4 raises instantly to the pressure of the fluid reservoir 7.

### Reference numeral

- 1: blood vessel catheter
- 2: first stop cock
- 3: temperature sensor
- 4: pressure sensor
- 5: first valve
- 6: second valve
- 7: reservoir
- 8: syringe
- 9: capillary
- 10: protection mechanism

- 11: second stop cock
- 12: automatic valve
- 13: check valve open by default
- 14: protection valve

- 21: first opening (injectate)
- 22: second opening (venous catheter)
- 23: third opening (pressure sensor)
- 24: spring
- 25: ball
- 26: sealing matter

## Claims

1. A device for detecting the start and end of injection during determination of cardio-circulatory parameters of a patient, comprising:
an injection channel for a blood vessel catheter (1) for injecting an injectate fluid into a blood vessel of the patient for carrying out thermodilution or other dilution measurements or other bolus injections in order to determine hemodynamic parameters of the patient, the injection channel comprising a pressure sensor (4) for sensing the pressure in the injection channel;
**characterized in that**
the pressure sensor (4) is configured to sense the central venous pressure of the patient between injection processes, and is also configured to sense a threshold of pressure in the injection channel as instants of begin and end of an injection process; and
a computer is connected to the pressure sensor (4) and configured to detect the threshold of pressure in the injection channel as the instants of begin and end of the injection process.

2. A device for detecting the start and end of injection during determination of cardio-circulatory parameters of a patient, comprising:
an injection channel for a blood vessel catheter (1) for injecting an injectate fluid into a blood vessel of the patient for carrying out thermodilution or other dilution measurements or other bolus injections in order to determine hemodynamic parameters of the patient, the injection channel comprising a pressure sensor (4) for sensing the pressure in the injection channel;
a protection mechanism (5, 6, 11, 12, 13, 14) associated with the pressure sensor (4) to protect the pressure sensor (4) against damages by high pressure during an injection process;
**characterized in that**
the pressure sensor (4) is configured to sense the central venous pressure of the patient between injection processes;
the protection mechanism (5, 6, 11, 12, 13, 14) of the pressure sensor (4) is configured to sense a threshold of pressure in the injection channel as instants of begin and end of the injection process; and
a computer is connected to the protection mechanism (5, 6, 11, 12, 13, 14) and configured to detect the threshold of pressure in the injection channel as the instants of begin and end of the injection process by monitoring an activation and deactivation of the protection mechanism (5, 11, 12, 13, 14).

3. The device according to any of the preceding claims, further comprising:
a syringe (8)
a first valve (5)
a second valve (6)
a temperature sensor (3) inside the injection channel for sensing the temperature of the injectate fluid passing through the injection channel, and
a reservoir (7) for injectate medium,
wherein from an upstream point to a downstream point the reservoir (7), the second valve (6), the first valve (5) and the temperature sensor are connected to each other by a fluid flow means;
the syringe (8) is connected to the fluid flow means between the first valve (5) and the second valve (6) and the pressure sensor (4) is arranged downstream the first valve (5).

4. The device according to any of the preceding claims further comprising:
a first stop cock (2) arranged downstream of all other components adapted to be connected to the catheter (1).

5. The device according to any of the preceding claims,
wherein the catheter (1) is a single lumen venous catheter.

6. The device according to claim 3,
wherein the pressure sensor (4) is additionally connected to the fluid flow means between the reservoir (7) and the second valve (6).

7. The device according to claim 2,
wherein the protection mechanism comprises a check valve (5) closed by default and a check valve (13) open by default.

8. The device according to claim 2,
wherein the protection mechanism comprises a check valve (5) closed by default and a protection valve (14) which closes at over pressure.

9. The device according to claim 2,
wherein the protection mechanism comprises an automatic valve (12).

10. The device as defined in claim 9, the automatic valve (12) comprising:
a first opening (21) to the injectate fluid,
a second opening (22) to the venous catheter (1) a third opening (23) to the pressure sensor (4),
a sealing matter (25 or 26) being movable between the first opening (21) and the third opening (23), and
a spring (24) extending to the first opening (21) and pressing the sealing
matter (25 or 26) against the first opening (21) thereby closing said first opening (21),
wherein upon a pressure over a predefined threshold being applied onto the first opening (21) by the injectate fluid the sealing matter (25 or 26) is pressed against the third opening (23) thereby giving free the first opening (21) and closing said third opening (23), and
wherein the automatic valve (12) is configured to comprise a stage within the movement of the sealing matter (25, 26) when the sealing matter (25, 26) is moved against the spring (24) and somewhat opens the pathway to the first opening (21), but where the sealing matter (25, 26) not fully closes the pathway of the third opening.

## Patentansprüche

1. Vorrichtung zum Detektieren von Beginn und Ende einer Injektion während der Bestimmung von Herz-Kreislauf-Parametern eines Patienten, umfassend:
einen Injektionskanal für einen Blutgefäßkatheter (1) zum Injizieren eines Injektionsfluids in ein Blutgefäß des Patienten im Hinblick auf die Durchführung von Thermodilutionsmessungen oder anderen Dilutionsmessungen oder für sonstige Bolusinjektionen zur Bestimmung hämodynamischer Parameter des Patienten, wobei der Injektionskanal einen Drucksensor (4) zum Erfassen des in dem Injektionskanal vorhandenen Drucks umfasst;
**dadurch gekennzeichnet, dass**
der Drucksensor (4) dafür ausgelegt ist, den zentralen Venendruck des Patienten zwischen Injektionsvorgängen zu erfassen, und außerdem dafür ausgelegt ist, einen jeweils in dem Injektionskanal vorhandenen Druckschwellenwert als Momente für Beginn und Ende eines Injektionsvorgangs zu erfassen; und
ein Computer mit dem Drucksensor (4) verbunden ist und dafür ausgelegt ist, den jeweils in dem Injektionskanal vorhandenen Druckschwellenwert als die Momente für Beginn und Ende des Injektionsvorgangs zu detektieren.

2. Vorrichtung zum Detektieren von Beginn und Ende einer Injektion während der Bestimmung von Herz-Kreislauf-Parametern eines Patienten, umfassend:
einen Injektionskanal für einen Blutgefäßkatheter (1) zum Injizieren eines Injektionsfluids in ein Blutgefäß des Patienten im Hinblick auf die Durchführung von Thermodilutionsmessungen oder anderen Dilutionsmessungen oder für sonstige Bolusinjektionen zur Bestimmung hämodynamischer Parameter des Patienten, wobei der Injektionskanal einen Drucksensor (4) zum Erfassen des in dem Injektionskanal vorhandenen Drucks umfasst;
einen Schutzmechanismus (5, 6, 11, 12, 13, 14), der dem Drucksensor (4) zugeordnet ist, um den Drucksensor (4) gegen Beschädigungen durch hohen Druck während eines Injektionsvorgangs zu schützen;
**dadurch gekennzeichnet, dass**
der Drucksensor (4) dafür ausgelegt ist, den zentralen Venendruck des Patienten zwischen Injektionsvorgängen zu erfassen;
der Schutzmechanismus (5, 6, 11, 12, 13, 14) des Drucksensors (4) dafür ausgelegt ist, einen jeweils in dem Injektionskanal vorhandenen Druckschwellenwert als Momente für Beginn und Ende des Injektionsvorgangs zu erfassen; und
ein Computer mit dem Schutzmechanismus (5, 6, 11, 12, 13, 14) verbunden ist und dafür ausgelegt ist, durch Überwachen einer Aktivierung und Deaktivierung des Schutzmechanismus (5, 11, 12, 13, 14) den jeweils in dem Injektionskanal vorhandenen Druckschwellenwert als die Momente für Beginn und Ende des Injektionsvorgangs zu detektieren.

3. Vorrichtung nach einem der vorangegangenen Ansprüche, ferner umfassend:
eine Injektionsspritze (8)
ein erstes Ventil (5)
ein zweites Ventil (6)
einen Temperatursensor (3) im Inneren des Injektionskanals zum Erfassen der Temperatur des durch den Injektionskanal strömenden Injektionsfluids, und
einen Vorratsbehälter (7) für Injektionsmittel,
wobei zwischen einer zuströmseitigen Stelle und einer abströmseitigen Stelle der Vorratsbehälter (7), das zweite Ventil (6), das erste Ventil (5) und der Temperatursensor durch ein Fluidstrommittel miteinander verbunden sind;
die Injektionsspritze (8) zwischen dem ersten Ventil (5) und dem zweiten Ventil (6) mit dem Fluidstrommittel verbunden ist und der Drucksensor (4) abströmseits von dem ersten Ventil (5) angeordnet ist.

4. Vorrichtung nach einem der vorangegangenen Ansprüche, ferner umfassend:
ein erstes Absperrventil (2), das abströmseits von allen anderen mit dem Katheter (1) verbindbaren Komponenten angeordnet ist.

5. Vorrichtung nach einem der vorangegangenen Ansprüche,
wobei es sich bei dem Katheter (1) um einen einlumigen Venenkatheter handelt.

6. Vorrichtung nach Anspruch 3,
wobei der Drucksensor (4) zusätzlich zwischen dem Vorratsbehälter (7) und dem zweiten Ventil (6) mit dem Fluidstrommittel verbunden ist.

7. Vorrichtung nach Anspruch 2,
wobei der Schutzmechanismus ein per Voreinstellung geschlossenes Rückschlagventil (5) und ein per Voreinstellung geöffnetes Rückschlagventil (13) umfasst.

8. Vorrichtung nach Anspruch 2,
wobei der Schutzmechanismus ein per Voreinstellung geschlossenes Rückschlagventil (5) und ein bei Überdruck schließendes Schutzventil (14) umfasst.

9. Vorrichtung nach Anspruch 2,
wobei der Schutzmechanismus ein Automatikventil (12) umfasst.

10. Vorrichtung nach Anspruch 9, wobei das Automatikventil (12) umfasst:
eine erste Öffnung (21) in Richtung zu dem Injektionsfluid,
eine zweite Öffnung (22) in Richtung zu dem Venenkatheter (1)
eine dritte Öffnung (23) in Richtung zu dem Drucksensor (4),
eine Dichtungsmasse (25 oder 26), welche zwischen der ersten Öffnung (21) und der zweiten Öffnung (23) beweglich ist, und
eine Feder (24), die sich zu der ersten Öffnung (21) hin erstreckt und die Dichtungsmasse (25 oder 26) gegen die erste Öffnung (21) drückt und dadurch die erste Öffnung (21) verschließt,
wobei, wenn ein durch das Injektionsfluid auf die erste Öffnung (21) ausgeübter Druck einen vordefinierten Schwellenwert übersteigt, die Dichtungsmasse (25 oder 26) gegen die dritte Öffnung (23) gedrückt wird, wodurch die erste Öffnung (21) freigegeben wird und die dritte Öffnung (23) verschlossen wird, und
wobei das Automatikventil (12) dafür ausgelegt ist, eine Phase in der Bewegung der Dichtungsmasse (25, 26) zu umfassen, in welcher die Dichtungsmasse (25, 26) gegen die Feder (24) bewegt wird und den Pfad in Richtung zu der ersten Öffnung (21) bis zu einem gewissen Grad öffnet, in welcher die Dichtungsmasse (25, 26) den Pfad in Richtung zu der dritten Öffnung jedoch nicht vollständig verschließt.

## Revendications

1. Dispositif permettant de détecter le début et la fin d'une injection pendant la détermination de paramètres cardio-vasculaires d'un patient, lequel dispositif comprend :
un canal d'injection pour un cathéter de vaisseau sanguin (1) permettant d'injecter un fluide d'injection dans un vaisseau sanguin du patient en vue de réaliser des mesures par thermodilution ou d'autres mesures par dilution, ou pour diverses injections en bolus destinées à déterminer des paramètres hémodynamiques du patient, le canal d'injection comprenant un capteur de pression (4) destiné à saisir la pression dans le canal d'injection ;
**caractérisé en ce que**
le capteur de pression (4) est conçu pour saisir la pression veineuse centrale du patient entre les processus d'injection et est en outre conçu pour saisir respectivement un seuil de pression dans le canal d'injection en tant qu'instants déterminant le début et la fin d'un processus d'injection ; et
un ordinateur est connecté au capteur de pression (4) et est conçu pour détecter respectivement le seuil de pression dans le canal d'injection en tant qu'instants déterminant le début et la fin dudit processus d'injection.

2. Dispositif permettant de détecter le début et la fin d'une injection pendant la détermination de paramètres cardio-vasculaires d'un patient, lequel dispositif comprend :
un canal d'injection pour un cathéter de vaisseau sanguin (1) permettant d'injecter un fluide d'injection dans un vaisseau sanguin du patient en vue de réaliser des mesures par thermodilution ou d'autres mesures par dilution, ou pour diverses injections en bolus destinées à déterminer des paramètres hémodynamiques du patient, le canal d'injection comprenant un capteur de pression (4) destiné à saisir la pression dans le canal d'injection ;
un mécanisme de protection (5, 6, 11, 12, 13, 14) qui est affecté au capteur de pression (4) afin de protéger ledit capteur de pression (4) contre des dommages occasionnés par une haute pression pendant un processus d'injection ;
**caractérisé en ce que**
le capteur de pression (4) est conçu pour saisir la pression veineuse centrale du patient entre des processus d'injection ;
le mécanisme de protection (5, 6, 11, 12, 13, 14) du capteur de pression (4) est conçu pour saisir respectivement un seuil de pression dans le canal d'injection en tant qu'instants déterminant le début et la fin du processus d'injection ; et
un ordinateur est connecté au mécanisme de protection (5, 6, 11, 12, 13, 14) et est conçu pour détecter respectivement le seuil de pression dans le canal d'injection en tant qu'instants déterminant le début et la fin dudit processus d'injection en surveillant une activation et désactivation du mécanisme de protection (5, 11, 12, 13, 14).

3. Dispositif selon l'une quelconque des revendications précédentes, lequel comprend en outre :
une seringue d'injection (8)
une première soupape (5)
une deuxième soupape (6)
un capteur de température (3) situé à l'intérieur du canal d'injection et destiné à saisir la température du fluide d'injection traversant le canal d'injection, et
un réservoir (7) pour le produit d'injection,
et dans lequel, entre un point situé en amont et un point situé en aval, le réservoir (7), la deuxième soupape (6), la première soupape (5) et le capteur de température sont reliés entre eux par un produit fluidique ;
la seringue d'injection (8) est reliée, entre la première soupape (5) et la deuxième soupape (6), au produit fluidique, et le capteur de pression (4) est disposé en aval de la première soupape (5).

4. Dispositif selon l'une quelconque des revendications précédentes, lequel comprend en outre :
un premier clapet d'arrêt (2) qui est disposé en aval de tous les autres composants qui peuvent être reliés au cathéter (1).

5. Dispositif selon l'une quelconque des revendications précédentes,
dans lequel le cathéter (1) utilisé est un cathéter veineux à une seule lumière.

6. Dispositif selon la revendication 3,
dans lequel le capteur de pression (4) est relié en outre, entre le réservoir (7) et la deuxième soupape (6), au produit fluidique.

7. Dispositif selon la revendication 2,
dans lequel le mécanisme de protection comprend un clapet anti-retour (5) fermé par défaut et un clapet anti-retour (13) ouvert par défaut.

8. Dispositif selon la revendication 2,
dans lequel le mécanisme de protection comprend un clapet anti-retour (5) fermé par défaut et un clapet de protection (14) se fermant en cas de surpression.

9. Dispositif selon la revendication 2,
dans lequel le mécanisme de protection comprend une soupape automatique (12).

10. Dispositif selon la revendication 9, dans lequel la soupape automatique (12) comprend :
une première ouverture (21) en direction du fluide d'injection,
une deuxième ouverture (22) en direction du cathéter veineux (1),
une troisième ouverture (23) en direction du capteur de pression (4),
une masse d'étanchéité (25 ou 26) qui peut être déplacée entre la première ouverture (21) et la deuxième ouverture (23), et
un ressort (24) qui s'étend vers la première ouverture (21) et presse la masse d'étanchéité (25 ou 26) contre la première ouverture (21), obturant ainsi la première ouverture (21),
dans lequel, lorsqu'une pression exercée par le fluide d'injection sur la première ouverture (21) dépasse une valeur de seuil prédéfinie, la masse d'étanchéité (25 ou 26) est pressée contre la troisième ouverture (23), ce qui libère la première ouverture (23) et obture la troisième ouverture (23), et
dans lequel la soupape automatique (12) est conçue pour comprendre une étape dans le mouvement de la masse d'étanchéité (25, 26) lors de laquelle ladite masse d'étanchéité (25, 26) est déplacée contre le ressort (24) et ouvre jusqu'à un certain degré la voie en direction de la première ouverture (21) et lors de laquelle la masse d'étanchéité (25, 26) obture, mais de manière incomplète, la voie en direction de la troisième ouverture.
